(19) Europäisches Patentamt
European Patent Office
Office européen des brevets

(11) Publication number: **0 525 888 A1**

(12) **EUROPEAN PATENT APPLICATION**

(21) Application number: **92202264.5**

(22) Date of filing: **23.07.92**

(51) Int. Cl.5: **C07C 59/68**, C07C 317/44, C07C 323/52, A61K 31/19

(30) Priority: **29.07.91 US 737098**

(43) Date of publication of application: **03.02.93 Bulletin 93/05**

(84) Designated Contracting States: **CH DE FR GB IT LI NL**

(71) Applicant: **MERCK & CO. INC.**
**126, East Lincoln Avenue P.O. Box 2000**
**Rahway New Jersey 07065-0900(US)**

(72) Inventor: **Witzel, Bruce E.**
**115 Scotch Plains Avenue**
**Westfield, NJ 07090(US)**
Inventor: **Tolman, Richard L.**
**29 Upper Warren Way**
**Warren, NJ 07059(US)**

(74) Representative: **Thompson, John Dr. et al**
**Merck & Co., Inc. European Patent**
**Department Terlings Park Eastwick Road**
**Harlow, Essex CM20 2OR(GB)**

(54) **New catechol type non-steroidal drugs as 5-alpha reductase inhibitors.**

(57) Described are new non-steroidal drugs for treatment of benign prostatic hyperplasia and other disorders mediated by high 5-alpha reductase activity, or high dihydrotestosterone levels, and other conditions of hyperandrogenic stimulation, of the formula:

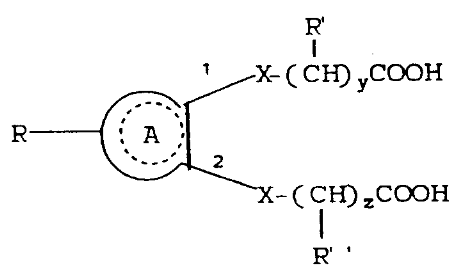

wherein
A, X, R, R', R'', y and z are as defined in claim 1.

EP 0 525 888 A1

## BACKGROUND OF THE INVENTION

It is well known in the art that certain undesirable physiological manifestations, such as acne vulgaris, seborrhea, female hirsutism, and male pattern baldness and particularly benign prostatic hypertrophy (BPH), art the result of hyperandrogenic stimulation caused by an excessive accumulation of testosterone or similar androgenic hormones in the metabolic system. Early attempts to provide a chemtherapeutic agent to counter the undesirable results of hyperandrogenicity resulted in the discovery of several steroidal antiandrogens having undesirable hormonal activities of their own. the estrogens, for example, not only counteract the effect of the androgens but can have a feminizing effect as well, e.g. a tendency to feminize a male host or the male fetus of a female host.

It more recently became known in the art that the principal mediator of androgenic activity in some target organs is 5α-dihydrotestosterone, and that it is formed locally in the target organ by the action of testosterone-5α-reductase or other 5α-reductase isozymes. It is known that various members of the 5α-reductase family of enzymes function to provide DHT levels in the control of various peripheral functions, i.e. male pattern baldness, beard growth, sebum production, etc. Any or all of these isozymic targets could be targets for a 5α-reductase inhibitor.

It therefore has been postulated and demonstrated that inhibitors of testosterone-5α-reductase will serve to prevent or lessen symptoms of hyperandrogenic stimulation. Nayfe et al., Steriods, 14, 269 (1969) demonstrated in vitro that methyl 4-androsten-3-one-17β-carboxylate was a testosterone-5α-reductase inhibitor. Then Voigt and Hsia, Endocrinology, 92, 1216 (1973), Canadian Pat. No. 970,692, demonstrated that the above ester and the parent free acid, 4-androsten-3-one-17β-carboxylic acid are both active inhibitors of testosterone-5α-reductase in vitro. They further demonstrated that topical application of either testosterone or 5α-dihydrotesterone caused enlargement of the female hamster flank organ, an androgen dependent sebaceous structure. However, concomitant administration of 4-androsten-3-one-17β-carboxylic acid or its methyl ester inhibited the response elicited by testosterone but did not inhibit the response elicited by 5α-dihydrotestosterone. These results were interpreted as indicating that the compounds were antiandrogenic by virtue of their ability to inhibit testosterone-5α-reductase.

Steroidal compounds particularly for the treatment of Benign Prostatic Hyperplasty (BPH) are well known in the art and constantly being developed. See the following patents: USP 4,317,817, USP 4,882,319 and EPO Publication Nos. 0 277 002, 0 343 954, 0 375 344, 0 375 345, 0 375 347, 0 375 349 (all assigned to SmithKline Beckmann Corporation) which disclose steroidal 5-alpha reductase inhibitors as BPH agents.

However, as described above, being steroids, they also can exhibit hormonal activity which may, in some cases, be undesirable, e.g. causing femininizing characteristics in men due to decreases in dihydrotestosterone.

Recent non-steroidal BPH active compounds have been disclosed in EP 291 245, EP 291 247 and EP 0 173 516 to ONO Pharmaceutical Co. However, these references do not describe catechol or thio-catechol type derivatives which are dicarboxylates and active versus 5α-reductase.

It is highly desired in the art to discover new non-steroidal BPH agents which do not possess steroidal activity with their attendant undesirable hormonal effects.

## SUMMARY OF THE INVENTION

By this invention is provided a new agent for the treatment of BPH of the following formula:

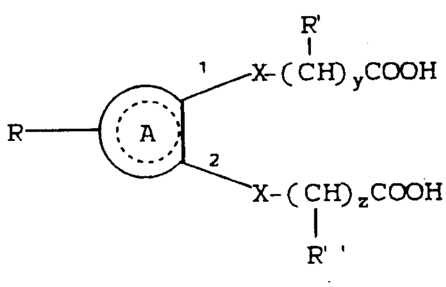

wherein
A is an 1,2-disubstituted aromatic ring; preferably a benzene ring;
wherein
X is independently O, S, SO, or $SO_2$;

| | |
|---|---|
| R is | H,<br>$C_1$-$C_4$ alkyl,<br>phenyl or substituted phenyl,<br>halo,<br>haloalkyl,<br>hydroxy,<br>carboxy,<br>cyano,<br>$C_1$-$C_4$ alkoxy,<br>$C_1$-$C_4$ alkylthio,<br>$C_1$-$C_4$ alkylsulfinyl,<br>$C_1$-$C_4$ alkylsulfonyl,<br>nitro,<br>amino,<br>$C_1$-$C_4$ mono or di-alkylamino; |
| R' and R'' are | independently<br>H,<br>halo,<br>$C_1$-$C_4$ alkyl or $C_1$-$C_4$ alkoxy,<br>amino, or oxo, where CH-R' or CH-R'' in the formula become -C=O; |
| y is | 1-6; |
| z is | 6-20; and<br>wherein |

$$\overset{R'}{\overset{|}{(CH)}}_y \text{ and } \overset{R''}{\overset{|}{(CH)}}_z$$

can independently represent substituted or unsubstituted alkyl radicals or alkenyl radicals containing at least one alkene bond;

and pharmaceutically acceptable salts and esters thereof.

The compounds of the instant invention are inhibitors of the human testosterone-5α-reductase enzyme.

## BRIEF DESCRIPTION OF THE INVENTION AND PREFERRED EMBODIMENTS

The scope of the compounds of the instant invention are described by the above-described formula.

In the description of the formula the following terms are used which are hereby defined:

X in the general formula above is O or S, preferably where one X is O, and particularly where both Xs are O, e.g., resulting in the catechol structure.

"$C_1$-$C_4$ alkyl" includes linear or branched species, e.g. methyl, ethyl, n-propyl, isopropyl, cyclopropyl, n-butyl, isobutyl, sec-butyl, t-butyl.

"$C_1$-$C_4$ alkoxy" includes linear or branched species, e.g., methoxy, ethoxy, n-propoxy, isopropoxy, n-butoxy, isobutoxy, sec-butoxy, t-butoxy.

"Halo" includes fluoro, chloro, bromo or iodo.

"Substituted phenyl" includes phenyl substituted by one or more of $C_1$-$C_4$ alkyl, $C_1$-$C_4$ alkoxy, or halo, and the like, as defined above. representative examples include o, m-, p-methoxy phenyl; 2,4-dimethoxyphenyl; 2-chloro-4-ethoxyphenyl; 3,5-dimethoxyphenyl; 2,4-dichlorophenyl; 2-bromo-4-methylphenyl, o-fluorophenyl, and the like.

"Haloalkyl" includes $C_1$-$C_4$ alkyl, defined above, substitued with one or more "halo" as defined above and includes: trifluoromethyl, 2,2-dichloroethyl and the like.

"$C_1$-$C_4$ alkylthio" includes $C_1$-$C_4$ alkyl, defined above, substituted with at least one divalent thio (-S-) grouping including; methylthio, ethylthio, isopropylthio, n-butylthio, and the like.

"$C_1$-$C_4$ alkylsulfinyl" includes $C_1$-$C_4$ alkyl, defined above, substituted with at least one -SO-grouping

including; methylsulfinyl, ethylsulfinyl; isopropylsulfinyl, and the like.

"$C_1$-$C_4$ alkylsulfonyl" includes $C_1$-$C_4$ alkyl, defined above, substituted with at least one sulfonyl group, $-SO_2-$, including; methylsulfonyl, ethylsulfonyl, isopropylsulfonyl, n-butylsulfonyl, and the like.

"$C_1$-$C_4$ mono or dialkyl amino" includes amino, substituted with one or more $C_1$-$C_4$ alkyl groups as defined hereinabove, including: methylamino, ethylamino, n-butylamino, t-butylamino, N,N-dimethylamino, N,N-diethylamino, methyl-t-butylamino, and the like.

The R group or groups on the benzene ring can be present initially in the process, such as in starting material I in Flow Chart A, e.g. phenyl, methyl, methoxy, cyano, trifluoromethyl, carbomethoxy, or added later by a conventional reaction, e.g. chloro, as by chlorination, nitro by nitration, or created from a starting or added functional group present, e.g. converting a nitro to an amino group by catalytic reduction, then alkylating to a mono or dialkylamine. An amino group can be subjected to diazotization to a hydroxy group, which can be followed by methylation to a methoxy group. Similarly, a hydroxy group can be converted to a thiol by the analogous procedures described in J. Org. Chem. 31, pp 3980-3984 (1966) by Newman and Karnes, and J. Org. Chem. 31, pp 410 (1966) by Kwart, H. and Evans, E.S. The resulting thiol can be alkylated to alkylthio, which can be oxidized to the corresponding sulfoxide or sulfone. Preferred substituents are H, $C_1$-$C_4$ alkyl, $C_1$-$C_4$ alkoxy and phenyl. These reactions and sequences are conventional in the art and it will be obvious to one skilled in the art to modify the benzene ring to arrive at an R radical disclosed herein.

By the term "pharmaceutically acceptable salts and esters thereof" is meant, salts and esters of the acid groups in the final molecule which can be used as part of the human drug delivery system and include the salts: sodium, potassium, calcium, ammonium, substituted ammonium, quaternary ammonium, and esters: ethyl ester, aceturate, besylate, edetate, phenpropionate, acetate, pamoate, and esters which serve as "prodrug" formulations which will hydrolyze in the body at physiological pH's to regenerate the acid, including pivaloylates, e.g. pivoxetil and pivoxil, and Kanebo esters, and the like.

$$(\overset{R'}{\overset{|}{C}}H)_y,$$

where y is 1-6, preferably 3, can contain at least one R' substituent as defined above, and can be, e.g., $-CH_2-$; $-CH_2-CH_2-$; $-CH_2-CH_2-CH_2-$,

$$-\underset{CH_3}{\underset{|}{CH}}-;\quad -\underset{CH_3}{\underset{|}{CH}}-CH_2-;\quad -CH_2-\underset{Cl}{\underset{|}{CH}}-;\quad -CH_2-\underset{OCH_3}{\underset{|}{CH}}-CH_2-;\quad -CH_2-\underset{OCH_2CH_3}{\underset{|}{CH}}-CH_2-;$$

and the like.

An alkene bond can also be present in

$$(\overset{R'}{\overset{|}{C}}H)_y-,$$

e.g., $CH_2-CH=CH-$; $CH_2-CH=CH-CH_2-$; $-CH_2-CH=CH-(CH_2)_2-$; and the like.

$$(\overset{R''}{\overset{|}{C}}H)_z,$$

where z is 6-20, preferably 10-16, can contain at least one R'' substituent as defined above, and can be; e.g., $-(CH_2)_6-$, $-(CH_2)_{20}-$,

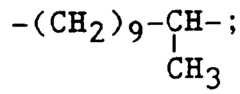

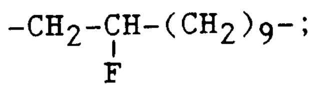 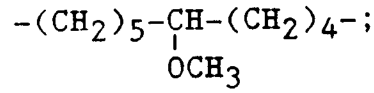

and the like.

An alkene bond can also be present in

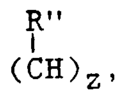

e.g., $CH_2$-CH = CH-$(CH_2)_8$-; -$(CH_2)_8$-CH = CH$(CH_2)_2$-; -$(CH_2)_9$-CH = CH-$(CH_2)_9$-; $(CH_2)_4$-CH = CH-$(CH_2)_4$-; and the like.

R' and R'' can also be -$NHCOCH_3$, which can be hydrolyzed to amino by conventional acid or base hydrolysis in the final molecule; R' and R'' can also be oxo, obtained by, for example, HBr addition to an alkene followed by conversion to an alcohol and subsequent oxidation to the ketone.

Preferred is where one R' or R'' is H and particularly preferred is where both

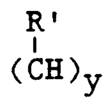

and

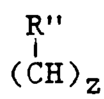

are alkyl.

Preferred compounds of the instant invention are given by the following formulas;

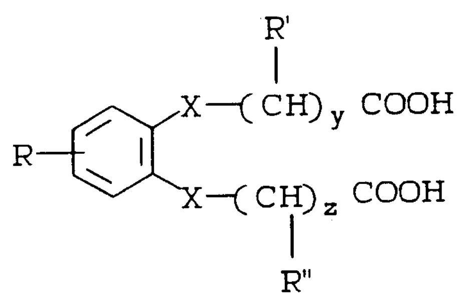

wherein

one X is O and R, R', R'', y and z are defined above; and particularly preferred are the compounds,

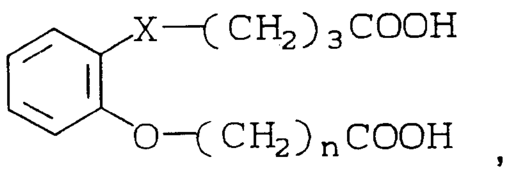

wherein
X is O or S and n is 10-16.

The compounds of the instant invention can be made by the procedure outlined in the following Flowchart A.

## FLOW CHART A

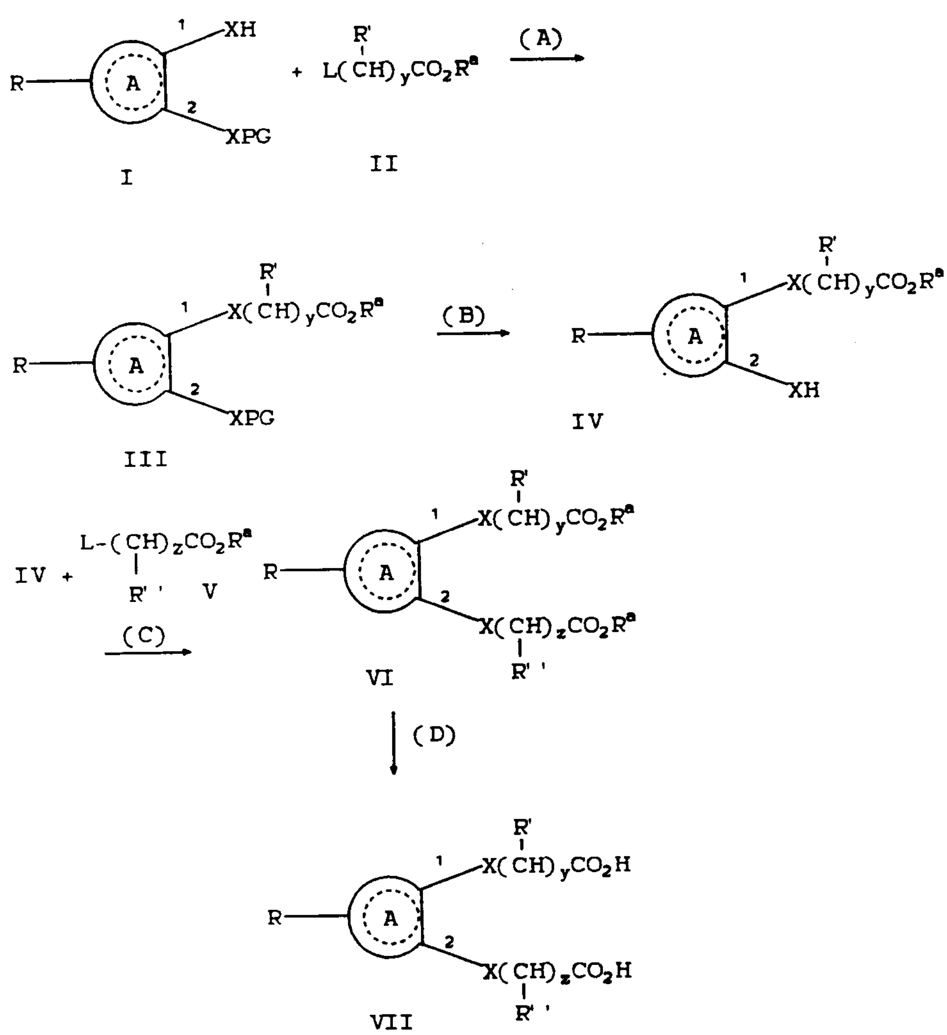


As seen in Flow Chart A, Compound I is the starting substrate in the invention process and is a 1,2-substituted benzene ring. X can be independently O or S and "PG" represents a hydroxy or thio protecting group which is inactive during Step (A) but can be subsequently removed by, e.g. palladium on carbon catalyst in ethanol under a pressurized $H_2$ atmosphere.

Examples of "PG" protecting groups which are conventional and known in the art (See "Protective

Groups in Organic Synthesis" by Theodora W. Greene - 1981 - John Wiley - Chapter 2, "Protection for the Hydroxyl Group, Including 1,2- and 1,3-Diols" pp. 16-87 and Chapter 6 "Protection for the Thiol Group" pp. 193-218).

Representative examples of "PG" include: benzyl, p-methoxybenzyl, p-halobenzyl; including p-chlorobenzyl, p-fluorobenzyl, and the like. Other protective groups which are known will be obvious to one skilled in the art to carry out the function of Step (A).

Representive examples of compounds useful as I in the instant invention, include, but are not limited to the following:

2-(benzyloxy)-phenol,
2-benzyloxy-thiophenol,
2-(benzylthio)-phenol,
2-(benzylthio)thiophenol,
3-methoxy-2-benzyloxyphenol,
2-benzyloxy-4-methoxyphenol,
3-methyl-2-benzyloxyphenol,
2-benzyloxy-5-methylphenol,
2-benzyloxy-4-methylphenol,
2-benzyloxy-5-methylphenol,
2-benzyloxy-3,5-diisopropylphenol,
2-benzyloxy-3,5-di-t-butylphenol,
2-benzyloxy-4-t-butylphenol,
2-benzyloxy-3-ethylphenol,
2-benzyloxy-5-phenylphenol,
2-benzyloxy-4-methyl-1-thiophenol,
2-benzyloxy-5-trifluoromethyl-1-thiophenol,
2-benzyloxy-6-methoxy-1-thiophenol,
2-benzylthio-4-methyl-thiophenol,
2-benzylthio-5-methylsulfonyl-phenol, and the like.

Representative examples of II useful in the invention process where L is a leaving group, e.g., halogen, including bromo, chloro, or sulfonate, and the like, and $R^a$ is a $C_1$-$C_4$ linear or branched alkyl portion of the ester, including methyl, ethyl, isopropyl, t-butyl, sec-butyl, and the like, and where $R^1$ and Y are or defined above, include, but are not limited to:

$Br-CH_2-COOMe$,
$Cl-CH_2CH_2CH_2COOCH_2CH_3$,
$Br-CH_2CH_2CH_2CH_2COOMe$,
$Br-CH_2CH_2CH_2CH_2CH_2COOEt$,
$Br-CH_2CH_2CH_2CH_2CH_2CH_2COOCH_2CH_2CH_2CH_3$,
$Br-(CH_2)_2CH(CH_3)COOMe$,
$Br-CH_2CH(CH_3)CH_2COOEt$,
$Br-CH_2CH_2CH_2COOMe$,
$Br-CH_2CH(OCH_3)CH_2COOCH(CH_3)_2$,
$Cl-CH_2CH(OCH_2CH_3)CH_2COOMe$,
$Br-CH_2CH(F)CH_2COOMe$,
$Cl-CH_2CH_2COOEt$,
and the like.

In Step (A) the condensation of I and II to produce III takes place in an non-hydroxylated polar organic solvent, e.g., acetone, ethyl acetate, methylethylketone, dioxan, THF, diethylketone, and the like, A proton acceptor is also present, e.g. potassium carbonate, sodium bicarbonate, pyridine, triethylamine, and the like. Generally, the reaction is carried out under an inert atomosphere, e.g. dry nitrogen, and heated at reflux or allowed to sit for an extended period of time at room temperature. Workup is conventional.

In Step (B) the protecting group, "PG", is catalytically removed at ambient temperature under a pressurized, hydrogen atmosphere in an organic solvent to produce IV, being a phenol or thiophenol. Operable catalysts include 5% Pd/C, and the like. The organic solvent should be inert under the reaction conditions and includes ethyl acetate, ethanol, methanol, dioxane, and the like.

Step (C) involves reacting IV with V to produce VI, the diester. The reaction conditions are similar to those described in Step (A) utilizing an inert organic solvent for the reactants and a proton acceptor.

Representative examples of V useful in the invention are:

$Br(CH_2)_6COOMe$,

$Br(CH_2)_7COOMe$,
$Br(CH_2)_8COOMe$,
$Br(CH_2)_9COOMe$,
$Br(CH_2)_{10}COOMe$,
$Br(CH_2)_{11}COOMe$,
$Cl(CH_2)_{12}COOEt$,
$Cl(CH_2)_{13}COOCH(CH_3)_2$,
$Cl(CH_2)_{14}COOCH_2CH_2CH_3$,
$Br\text{-}(CH_2)_{15}COOMe$
$Br\text{-}(CH_2)_{16}COOMe$
$Br(CH_2)_{16}COOCH_2CH_3$,
$Br(CH_2)_{17}COOC(CH_3)_3$,
$Br(CH_2)_{18}COOMe$,
$Br(CH_2)_{19}COOEt$,
$Br(CH_2)_{20}COOMe$,
$Br(CH_2)_2CH(CH_3)\text{-}(CH_2)_{10}COOMe$,
$Br\text{-}CH_2CH(CH_3)(CH_2)_{10}COOMe$,
$Br\text{-}CH_2CH_3CH(CH_3)CH_2COOEt$,
$Br\text{-}CH_2CH(OCH_3)(CH_2)_7COOCH(CH_3)_2$,
$Cl\text{-}CH_2CH(OCH_2CH_3)CH_2CH_2COOMe$,
$Br\text{-}CH_2CH(NHCOCH_3)\text{-}(CH_2)_{10}\text{-}CH_2\text{-}COOMe$,

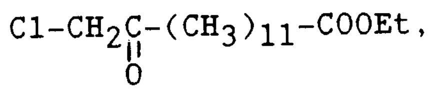

$Br\text{-}CH_2\text{-}CH=CH(CH_2)_8\text{-}COOEt$,

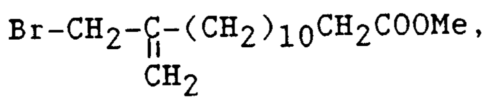

$Br\text{-}CH_2\text{-}(CH_2)_9\text{-}CH=CH\text{-}COOMe$,
and the like.

In Step (D), the diester can be deesterified by aqueous basic hydrolysis, e.g. NaOH in $MeOH/H_2O$ to yield the diacid VII upon acidification.

## FLOW CHART B

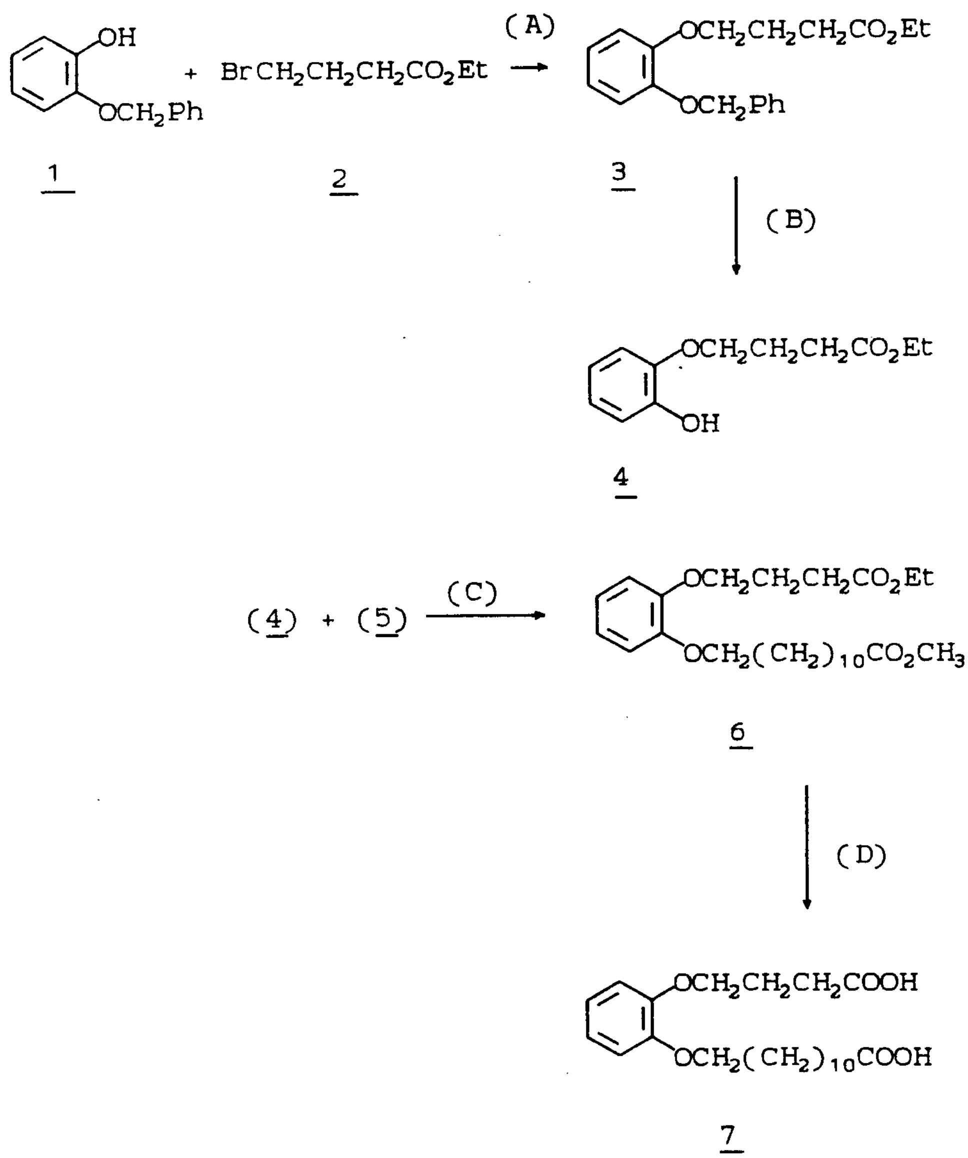


1 2 3 4 6 7

## FLOW CHART C

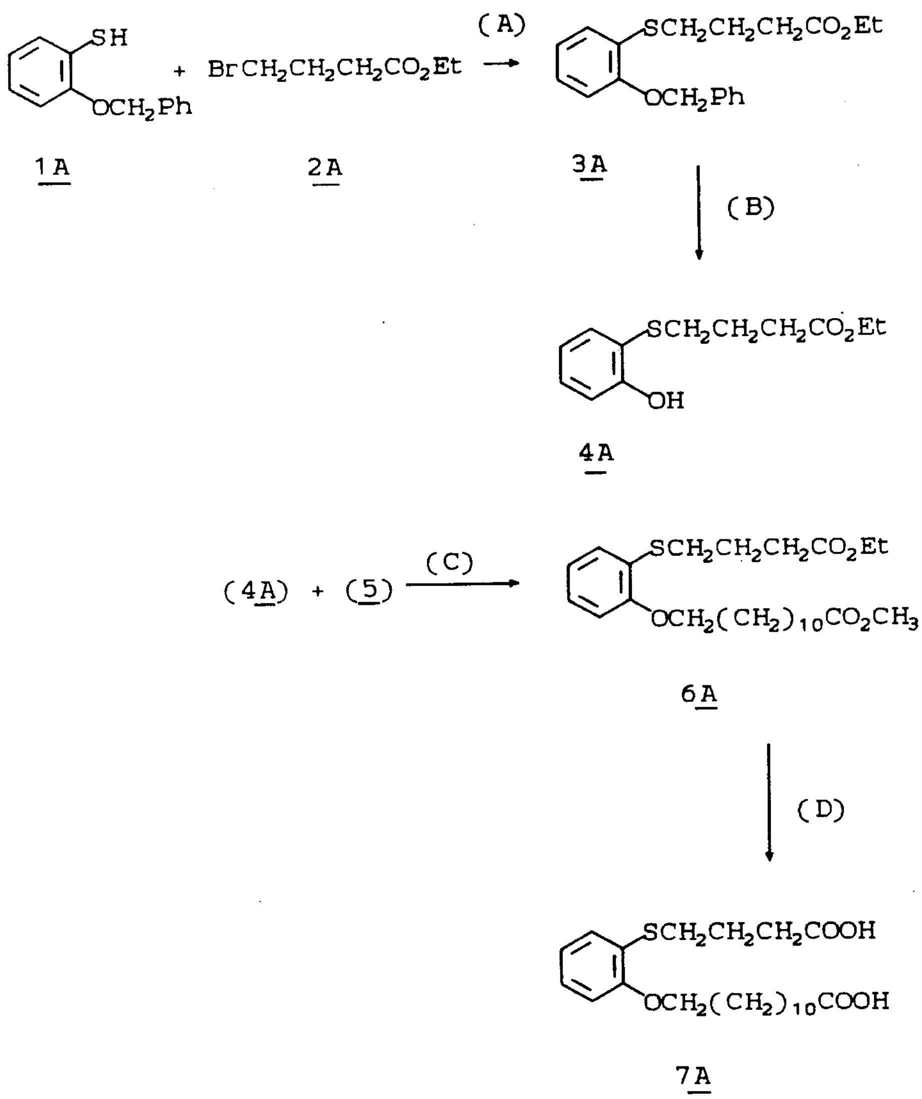


1A 2A 3A 4A 6A 7A

Flow Sheet B illustrates the specific synthesis of 7.

As seen, 2-benzyloxyphenol 1, ethyl 4-bromobutyrate 2 and anhydrous $K_2CO_3$ in e.g., dry acetone are heated at reflux or stirred for an extended period of time at room temperature, under a nitrogen atmosphere to give product ethyl 4-(2-benzyloxyphenoxy) butyrate 3, in Step (A).

A solution of 3 in e.g., ethyl acetate is catalytically hydrogenated at room temperature under e.g. 40 psig of $H_2$ in the presence of a 5% Pd/C catalyst to yield ethyl 4-(2-hydroxyphenoxy) butyrate 4 in Step (B).

Step (C) comprises reacting 4 and methyl 12-bromododecanoate 5 with potassium carbonate in acetone as in Step (A) to obtain the monomethyl ester 6.

In Step (D), the diester 6 is de-esterified by e.g., 2.5 N NaOH in MeOH/$H_2O$ to yield the final product, diacid 7,upon acidification.

Flow Sheet C illustrates the synthesis of the sulfur analog of 7 as 7A. This analogous procedure uses substantially the same steps as involved in Flow Sheet B.

It is also obvious from the above Flow Sheets that suitable replacement compounds for II and 2 with other substituted and unsubstituted halo alkyl esters, known in the art and described herein, and that suitable replacement of V and 5 with other bromoesters, available in the art and also described above will yield all of the compounds within the scope of the instant claims.

Representative examples of compounds produced by this process include:

4-(2-(20-Carboxyeicosyloxy)phenoxy)butyric acid;
4-(2-(19-Carboxynonadecyloxy)phenoxy)butyric acid;
4-(2-(18-Carboxyoctadecyloxy)phenoxy)butyric acid;
4-(2-(17-Carboxyheptadecyloxy)phenoxy)butyric acid;
4-(2-(16-Carboxyhexadecyloxy)phenoxy)butyric acid;
4-(2-(15-Carboxypentadecyloxy)phenoxy)butyric acid;
4-(2-(14-Carboxytetradecyloxy)phenoxy)butyric acid;
4-(2-(13-Carboxytridecyloxy)phenoxy)butyric acid;
4-(2-(12-Carboxydodecyloxy)phenoxy)butyric acid;
4-(2-(11-Carboxyundecyloxy)phenoxy)butyric acid;
4-(2-(10-Carboxydecyloxy)phenoxy)butyric acid;
4-(2-(9-Carboxynonyloxy)phenoxy)butyric acid;
4-(2-(8-Carboxyoctyloxy)phenoxy)butyric acid;
4-(2-(7-Carboxyheptyloxy)phenoxy)butyric acid;
4-(2-(6-Carboxyhexyloxy)phenoxy)butyric acid;
4-(2-(20-Carboxyeicosyloxy)phenylthio)butyric acid;
4-(2-(19-Carboxynonadecyloxy)phenylthio)butyric acid;
4-(2-(18-Carboxyoctadecyloxy)phenylthio)butyric acid;
4-(2-(17-Carboxyheptadecyloxy)phenylthio)butyric acid;
4-(2-(16-Carboxyhexadecyloxy)phenylthio)butyric acid;
4-(2-(15-Carboxypentadecyloxy)phenylthio)butyric acid;
4-(2-(14-Carboxytetradecyloxy)phenylthio)butyric acid;
4-(2-(13-Carboxytridecyloxy)phenylthio)butyric acid;
4-(2-(12-Carboxydodecyloxy)phenylthio)butyric acid;
4-(2-(11-Carboxyundecyloxy)phenylthio)butyric acid;
4-(2-(10-Carboxydecyloxy)phenylthio)butyric acid;
4-(2-(9-Carboxynonyloxy)phenylthio)butyric acid;
4-(2-(8-Carboxyoctyloxy)phenylthio)butyric acid;
4-(2-(7-Carboxyheptyloxy)phenylthio)butyric acid;
4-(2-(6-Carboxyhexyloxy)phenylthio)butyric acid;
4-(2-(20-Carboxyeicosylthio)phenoxy)butyric acid;
4-(2-(19-Carboxynonadecylthio)phenoxy)butyric acid;
4-(2-(18-Carboxyoctadecylthio)phenoxy)butyric acid;
4-(2-(17-Carboxyheptadecylthio)phenoxy)butyric acid;
4-(2-(16-Carboxyhexadecylthio)phenoxy)butyric acid;
4-(2-(15-Carboxypentadecylthio)phenoxy)butyric acid;
4-(2-(14-Carboxytetradecylthio)phenoxy)butyric acid;
4-(2-(13-Carboxytridecylthio)phenoxy)butyric acid;
4-(2-(12-Carboxydodecylthio)phenoxy)butyric acid;
4-(2-(11-Carboxyundecylthio)phenoxy)butyric acid;
4-(2-(10-Carboxydecylthio)phenoxy)butyric acid;
4-(2-(9-Carboxynonylthio)phenoxy)butyric acid;
4-(2-(8-Carboxyoctylthio)phenoxy)butyric acid;
4-(2-(7-Carboxyheptylthio)phenoxy)butyric acid;
4-(2-(6-Carboxyhexylthio)phenoxy)butyric acid;
4-(2-(20-Carboxyeicosylthio)phenylthio)butyric acid;
4-(2-(19-Carboxynonadecylthio)phenylthio)butyric acid;
4-(2-(18-Carboxyoctadecylthio)phenylthio)butyric acid;
4-(2-(17-Carboxyheptadecylthio)phenylthio)butyric acid;
4-(2-(16-Carboxyhexadecylthio)phenylthio)butyric acid;
4-(2-(15-Carboxypentadecylthio)phenylthio)butyric acid;
4-(2-(14-Carboxytetradecylthio)phenylthio)butyric acid;
4-(2-(13-Carboxytridecylthio)phenylthio)butyric acid;
4-(2-(12-Carboxydodecylthio)phenylthio)butyric acid;
4-(2-(11-Carboxyundecylthio)phenylthio)butyric acid;
4-(2-(10-Carboxydecylthio)phenylthio)butyric acid;
4-(2-(9-Carboxynonylthio)phenylthio)butyric acid;

4-(2-(8-Carboxyoctylthio)phenylthio)butyric acid;
4-(2-(7-Carboxyheptylthio)phenylthio)butyric acid;
4-(2-(6-Carboxyhexylthio)phenylthio)butyric acid;
3-(2-(16-Carboxyhexadecyloxy)phenoxy)propionic acid;
3-(2-(15-Carboxyisohexadecyloxy)phenoxy)butyric acid;
3-(2-(14-Carboxytetradecyloxy)phenoxy)butyric acid;
5-(2-(13-Carboxytridecyloxy)phenoxy)valeric acid;
5-(2-(12-Carboxydodecyloxy)phenoxy)valeric acid;
5-(2-(11-Carboxyisododecyloxy)phenoxy)valeric acid;
4-(2-(11-Carboxyundecyloxy)phenoxy)valeric acid;
4-(2-(10-Carboxydecyloxy)phenoxy)valeric acid;
4-(2-(9-Carboxynonyloxy)phenoxy)valeric acid;
6-(2-(9-Carboxynonyloxy)phenoxy)caproic acid;
6-(2-(8-Carboxyoctyloxy)phenoxy)caproic acid;
6-(2-(7-Carboxyisooctyloxy)phenoxy)caproic acid;
7-(2-(7-Carboxyheptyloxy)phenoxy)enanthic acid;
7-(2-(6-Carboxyhexyloxy)phenoxy)enanthic acid;
7-(2-(5-Carboxyisohexyloxy)phenoxy)enanthic acid;
2-(2-(12-Carboxydodecylthio)phenoxy)acetic acid;
2-(2-(11-Carboxydecylthio)phenoxy)acetic acid;
2-(2-(10-Carboxydecylthio)phenoxy)acetic acid;
3-(2-(9-Carboxynonyloxy)phenylthio)propionic acid; 3-(2-(12-Carboxydodecyloxy)phenylthio)propionic acid;
3-(2-(11-Carboxyundecyloxy)phenylthio)propionic acid;
3-(2-(11-Carboxyundecyloxy)phenylthio)butyric acid;
3-(2-(11-Carboxyundecylthio)-4-methyl-phenylthio) butyric acid;
3-(2-(12-Carboxydodecylthio)phenylthio)butyric acid;
5-(2-(11-Carboxyundecylthio)phenylthio)valeric acid;
5-(2-(10-Carboxydecyloxy)phenylthio)valeric acid;
5-(2-(9-Carboxynonyloxy)phenylthio)valeric acid;
3-(2-(12-Carboxydodecyloxy)phenylthio)valeric acid;
3-(2-(11-Carboxydecyloxy)phenylthio)valeric acid;
3-(2-(10-Carboxydecyloxy)phenylthio)valeric acid;
6-(2-(9-Carboxynonylthio)phenylthio)caproic acid;
6-(2-(12-Carboxydodecyloxy)phenylthio)caproic acid;
6-(2-(11-Carboxyundecyloxy)phenylthio)caproic acid;
6-(2-(11-Carboxyundecyloxy)-3-methylphenylthio)enanthic acid;
7-(2-(11-Carboxyundecyloxy)-4-methylphenylthio)enanthic acid;
7-(2-(12-Carboxydodecylthio)phenoxy)enanthic acid;
4-(2-(11-Carboxyundecyloxy)4-methyl-phenoxy)butyric acid;
4-(2-(10-Carboxydecyloxy)3-methylphenoxy)butyric acid;
4-(2-(9-Carboxynonyloxy)5-methylphenoxy)butyric acid;
4-(2-(12-Carboxydodecyloxy)6-methylphenoxy)butyric acid;
4-(2-(12-Carboxydodecyloxy)6-methylphenoxy)butyric acid;
4-(2-(11-Carboxyundecylthio)-3-(methylthio)phenoxy)valeric acid;
4-(2-(11-Carboxyundecylthio)-3-(methylsulfonyl) phenoxy)butyric acid;
4-(2-(11-Carboxyundecyloxy)-4-(methylsulfonyl) phenoxy)butyric acid;
4-(2-(12-Carboxydodecyloxy)5-ethyl-phenoxy)butyric acid;
4-(2-(11-Carboxyundecyloxy)4-phenylphenoxy)butyric acid;
4-(2-(10-Carboxydecyloxy)-3,5-dimethylphenoxy)butyric acid;
4-(2-(9-Carboxynonyloxy)-4-fluoro-phenoxy)butyric acid;
4-(2-(12-Carboxydodecyloxy)-5-(trifluoromethyl)phenoxy)butyric acid;
4-(2-(12-Carboxydodecylthio)-5-nitrophenoxy)butyric acid;
4-(2-(11-Carboxyundecylthio)-4-methylphenoxy)valeric acid;
4-(2-(11-Carboxyundecylthio)-3,5-di-methylphenoxy)butyric acid;
4-(2-(12-Carboxydodecyloxy)-4-(dimethylamino)phenoxy)butyric acid;
4-(2-(11-Carboxyundecyloxy)-5-(ethylamino)phenoxy)butyric acid;
2-(2-(9-Carboxynonyloxy)phenoxy)propionic acid;
3-(2-(12-Carboxydodecyloxy)phenoxy)-3-methylpropionic acid;

4-(2-(10-Carboxydecyloxy)phenylthio)-3-methoxy-butyric acid;
4-(2-(9-Carboxynonyloxy)phenylthio)-3-ethoxy-butyric acid;
4-(2-(11-Carboxyundecyloxy)phenoxy)but-2-enoic acid;
4-(2-(9-Carboxynonyloxy)phenoxy)-2-butenoic acid;
4-(2-(11-Carboxy-2-methylundecyloxy)phenoxy)butyric acid;
4-(2-(11-Carboxyundecyl-7-ene-oxy)phenoxy)butyric acid;
4-(2-(13-Carboxy-2-methylene-tri-decyloxy)phenoxy)butyric acid;
4-(2-(11-Carboxyundecyloxy)phenylsulfonyl)butyric acid
4-(2-(11-Carboxyundecyloxy)phenylsulfinyl)butyric acid
4-(2-(11-Carboxyundecylsulfinyl)phenoxy)butyric acid
4-(2-(11-Carboxyundecylsulfonyl)phenoxy)butyric acid
4-(2-(11-Carboxyundecylsulfinyl)phenylsulfinyl)butyric acid
4-(2-(11-Carboxyundecylsulfonyl)phenylsulfonyl)butyric acid
and the like.

The compounds of the present invention, prepared in accordance with the method described above, are, as already described, potent antiandrogens by virtue of their ability to specifically inhibit testosterone-5α-reductase.

Accordingly, the present invention is also particularly concerned with providing a method of treating the hyperandrogenic conditions of acne vulgaris, seborrhea, and female hirsutism by topical administration, and a method of treating all of the above conditions as well as benign prostatic hypertrophy, by oral or parenteral administration, of the novel compounds of the present invention.

The present invention is thus also concerned with providing suitable topical, oral and parenteral pharmaceutical formulations for use in the novel methods of treatment of the present invention.

The compositions containing the compounds of the present invention as the active ingredient for use in the treatment of 5-alpha reductase disorders including benign prostatic hyperplasia can be administered in a wide variety of therapeutic dosage forms in conventional vehicles for systemic administration, as, for example, by oral administration in the form of tablets, capsules, solutions, or suspensions, of by intravenous injection. The daily dosage of the products may be varied over a wide range varying from 50 to 2,000 mg. The compositions are preferably provided in the form of scored tablets containing 5, 10, 25, 50, 100, 150, 250, and 500 milligrams of the active ingredient for the symptomatic adjustment of the dosage to the patient to be treated. A therapeutically effective amount of the drug is ordinarily supplied at a dosage level of from about 0.01 mg to about 50 mg/kg of body weight per day. Preferably the range is from about 0.1 mg to 7 mg/kg of body weight per day. These dosages are well below the toxic dose of the product. Capsules containing the product of this invention can be prepared by mixing an active compound of the present invention with lactose and magnesium stearate, calcium stearate, starch, talc, or other carriers, and placing the mixture in gelating capsule. Tablets may be prepared by mixing the active ingredient with conventional tableting ingredients such as calcium phosphate, lactose, corn starch or magnesium stearate. The liquid forms in suitably flavored suspending or dispersing agents such as the synthetic and natural gums, for example, tragacanth, acacia, methylcellulose and the like. Other dispersing agents which may be employed include glycerin and the like. For parenteral administration, sterile suspensions and solutions are desired. Isotonic preparations which generally contain suitable preservative are employed when intravenous administration is desired.

For the treatment of acne vulgaris, seborrhea, female hirsutism, the compounds of the present invention are administered in the formula of pharmaceutical composition comprising the active compound in combination with a pharmacologically acceptable carrier adpated for topical administration. These topical pharmaceutical compositions may be in the form of a cream, ointment, gel or aerosol formulation adapted for application to the skin. These topical pharmaceutical compositions containing the compounds of the present invention ordinarily include about 0.1% to 15%, preferably about 5%, of the active compounds, in admixture with about 95% of vehicle.

The method of preparing the novel compounds of the present invention, already described above in general terms, may be further illustrated by the following examples which should not be construed as being limitations on the scope or spirit of the instant invention.

EXAMPLE 1

Synthesis of 4-(2-(11-Carboxyundecyloxy)phenoxy)butyric acid (7)

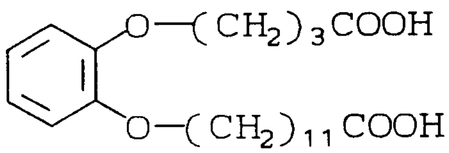

7

A. Ethyl 4-(2-Benzyloxyphenoxy)-butyrate (3)

To a stirred solution of 2-benzyloxyphenol (1) (4.0 g, 20 nM) and ethyl 4-bromobutyrate (2) (5.6 g, 28.7 mM) in dried acetone (100 mL) was added anhydrous ground potassium carbonate (6.0 g, 44 mM) and the resultant mixture heated at reflux under nitrogen until TLC analysis showed the absence of the starting phenol. Flash chromatography (silica gel, 15% ethyl acetate/hexane as eluant) of the filtered and concentrated mixture yielded 3.0 g of product (3) as a clear oil.

When the ethyl 4-bromobutyrate is replaced by halo-esters in the above example, the corresponding ether-ester is obtained. Likewise, when the above phenol is replaced by other substituted 2-benzyloxyphenols, the corresponding 2-substituted ethers are obtained. Substitution of a 2-benzyloxyor a 2-benzylthio-thiophenol for the 2-benzyloxyphenol yields the corresponding thioether-ester.

B. Ethyl 4-(2-Hydroxyphenoxy)-butyrate (4)

A mixture of (3) (1.57 g, 5.0 mM), ethanol (50 mL), glacial acetic acid (7 drops) and 10% palladium on carbon (0.7 g) was reacted in a hydrogen atmosphere (40 p.s.i.) at room temperature until hydrogen uptake ceased. Concentration of the filtered mixture yielded the product (4) as an oil.

C. Ethyl 4-(2-11-(Carbomethoxy) undecyloxyphenoxy)butyrate (6)

When (4) (0.224 g, 1.0 mM) and methyl 12-bromododecanoate (5) (0.32 g, 1.1 mM) were reacted with potassium carbonate in acetone as per Example (A), there was obtained product 6 (0.3 g) as a colorless oil.

When methyl 12-bromododecanoate is replaced by other halo esters in the above example, the corresponding diester is obtained.

D. 4-(2-(11-Carboxyundecyloxy)phenoxy)butyric acid (7)

To a stirred solution of (6) (0.102 g, 0.23 mM) in methanol (4 mL) and water (3 drops) was added 2.5 N sodium hydroxide solution (0.55 mL, 1.37 mM) dropwise, and the resultant mixture cleared with additional methanol (2 mL). When TLC analysis (2% methanol in methylene chloride (10 mL) containing glacial acetic acid (4 drops) indicated no mono- or diester remained, the methanol was removed in vacuo, the residue stirred with water (10 mL), and the solution filtered and acidified with 2N hydrochloric acid. Filtration of the resultant precipitate followed by washing with water and drying yielded product 7 (88 mg) as a white solid; M.P. collapses at 95 ° C, all melted at 105 ° C (uncorr.; A. O. Spencer Hot Stage).

| Calculated for $C_{22}H_{34}O_6$: | | |
|---|---|---|
| | C. 66.98; | H. 8.69. |
| Found: | C. 67.32; | H. 8.45. |

Compounds (1)-(7) all had NMR and Mass Spectral data consistant with their assigned molecular formulas.

4-(2-(11-Carboxyundecyloxy) phenylsulfinyl)butyric acid(7B)

When the subject thioethers, e.g., Compound 7A in Flow Sheet C, as the ester or acid, are treated with sodium metaperoidate in a suitable solvent (e.g., acetone/water) the corresponding sulfoxides are obtained. Likewise, reaction of the subject thioethers with m-chloroperbenzoic acid yields the corresponding sulfones.

For example, when 4-(2-(11-carboxyundecyloxy)phenylthio butyric acid 7A (0.41 g, 1.0 mM) in acetone (25 mM) is reacted with sodium metaperiodate (0.72 g, 3.3 mM) in water at room temperature, the title sulfoxide 7B is obtained. When the same starting material in methylene chloride is reacted with excess m-chlorobenzoic acid, the corresponding sulfone is obtained.

## Claims

**1.** A compound of the structure:

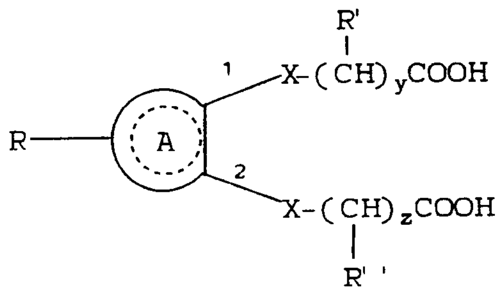

wherein
A is an 1,2-disubstituted aromatic ring;
wherein

| | |
|---|---|
| X is | independently O, S, SO, or $SO_2$; |
| R is | H,<br>$C_1$-$C_4$ alkyl,<br>phenyl or substituted phenyl,<br>halo,<br>haloalkyl,<br>hydroxy,<br>carboxy,<br>cyano,<br>$C_1$-$C_4$ alkoxy,<br>$C_1$-$C_4$ alkylthio,<br>$C_1$-$C_4$ alkylsulfinyl,<br>$C_1$-$C_4$ alkylsulfonyl,<br>nitro,<br>amino,<br>$C_1$-$C_4$ mono or di-alkylamino; |
| R' and R" | are independently<br>H,<br>halo,<br>$C_1$-$C_4$ alkyl or $C_1$-$C_4$ alkoxy,<br>amino, or oxo, where CH-R' or CH-R" in the formula become -C=O; |
| y is | 1-6; |
| z is | 6-20; and<br>wherein |

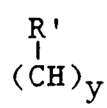

and

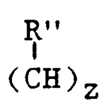

can independently represent substituted or unsubstituted alkyl or alkenyl radicals containing at least one alkene bond;

and pharmaceutically acceptable salts and esters thereof.

**2.** The compound of claim 1 wherein one X is 0.

**3.** The compound of claim 1 wherein R is H, phenyl, $C_1$-$C_4$ alkyl, $C_1$-$C_4$ alkoxy or phenyl.

**4.** The compound of claim 1 wherein one of R' and R'' are H.

**5.** The compound of claim 1 wherein y is 3.

**6.** The compound of claim 1 wherein (Z) is 10-16.

**7.** The compound of claim 1 wherein both

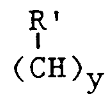

and

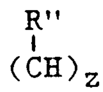

are alkyl.

**8.** The compound of claim 1 of the structure:

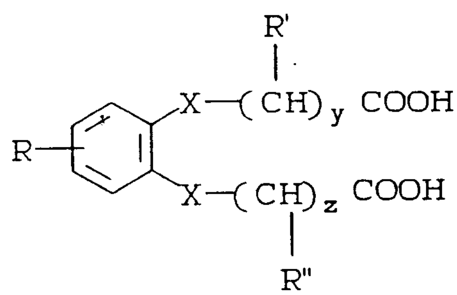

wherein

one X is 0;

R is H, , $C_1$-$C_4$ alkyl, phenyl or substituted phenyl, halo, haloalkyl, hydroxy, $C_1$-$C_4$ alkoxy, $C_1$-$C_4$ alkylthio, $C_1$-$C_4$ alkylsulfinyl, $C_1$-$C_4$ alkylsulfonyl, amino, $C_1$-$C_4$ mono or di-alkylamino;

R' and R'' are H;

y is 1-6; z is 6-20.

**9.** The use of a compound of Claim 1 for the manufacture of a medicament for treating the hyperandrogenic condition of acne vulgaris, seborrhea, female hirsutism, and benign prostatic hyperplasia.

**10.** A pharmaceutical composition comprising a pharmaceutically acceptable carrier and a therapeutically effective amount of a compound according to Claim 1.

European Patent Office

# EUROPEAN SEARCH REPORT

Application Number

EP 92 20 2264

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 032 063 (WARNER-LAMBERT) *Complete specification* | 1-10 | C07C59/68<br>C07C317/44<br>C07C323/52<br>A61K31/19 |
| A | CHEMICAL ABSTRACTS, vol. 76, no. 20, 15 May 1972, Columbus, Ohio, US; abstract no. 113624t, KOLESNIKOV G.S. ET. AL. 'Kinetics of the polycondensation of salts of ether dicarboxylic acids with aliphatic diamines studied by dynamic thermogravimetric analysis.' page 8 ;column R ; * abstract * & VYSOKOMOL. SOEDIN., SERIE B vol. 13, no. 11, 1971, MOSCOW pages 851 - 855 | 1-8 | |
| D,A | EP-A-0 291 245 (ONO PHARMACEUTICAL) *Page 31-37: claims.* | 1-10 | |
| | | | TECHNICAL FIELDS SEARCHED (Int. Cl.5 ) |
| | | | C07C |

The present search report has been drawn up for all claims

| Place of search | Date of completion of the search | Examiner |
|---|---|---|
| THE HAGUE | 03 NOVEMBER 1992 | LUYTEN H.W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)